# EUROPEAN PATENT SPECIFICATION

(11) **EP 1 702 575 B1**
(45) Date of publication and mention of the grant of the patent: **13.05.2009**
(21) Application number: 06005056.4
(22) Date of filing: 13.03.2006
(51) Int. Cl.: A61B 17/34, A61B 17/00, A61M 25/06, A61B 19/00

(54) **Radially expandable access system including trocar seal**
Radial expandierbares Zugangssystem mit Trokardichtung
Système d'accès expansible radialement avec un joint de trocart

(30) Priority: 16.03.2005 US 81766
(43) Date of publication of application: 20.09.2006
(73) Proprietor: Tyco Healthcare Group LP, Norwalk, Connecticut 06856 (US)
(72) Inventor: Farascioni, David, Bethel, CT 06801 (US)
(74) Representative: HOFFMANN EITLE

(56) References cited:
- WO-A-03/071926
- US-A- 5 431 676
- US-A- 5 643 282
- US-A- 5 797 888
- US-A1- 2004 199 121
- US-B1- 6 530 923

## Description

### BACKGROUND

### Technical Field

The present disclosure relates generally to apparatus and methods for providing access to an internal operative site during a surgical procedure and, more particularly, to access systems which may be percutaneously or otherwise introduced while in a narrow diameter configuration and which after introduction may be radially expanded to accommodate passage of larger diameter surgical instruments therethrough.

### Background of Related Art

Minimally invasive surgical procedures rely on obtaining percutaneous access to an internal surgical site using small-diameter access tubes (typically 5 to 12 mm), usually referred to as trocars, which penetrate through the skin and which open to the desired surgical site. A viewing scope is then introduced through one such trocar, and the surgeon operates using instruments introduced through other appropriately placed trocars while viewing the operative site on a video monitor connected to the viewing scope. The surgeon is thus able to perform a wide variety of surgical procedures requiring only several 5mm to 12 mm punctures at the surgical site. As a result, patient trauma and recovery time are typically reduced.

Particular minimally invasive surgical procedures are often referred to based on the type of scope used to view the region of the body which is the operative site. For example, procedures in the abdominal area, which rely on a laparoscope for viewing, are typically referred to as laparoscopic procedures. In such laparoscopic procedures, the patient's abdominal region is typically insufflated (filled with pressured gas) to raise the abdominal wall and create sufficient operating space to perform a desired procedure. The trocars used in laparoscopic procedures must therefore include a valve at their proximal end to allow passage of the scope or surgical instruments while inhibiting leakage of the insufflating gas. It has also been proposed to perform laparoscopic procedures by mechanically expanding the abdomen rather than using insufflation.

Recently, a radially expandable access system has been developed, as shown and described in U.S. Pat. Nos. 5,183,464; 5,431,676; 5,814,058; 5,827,319; 6,080,174; 6,245,052; 6,325,812; 6,494,893; and 6,589,225, as well as in U.S. Pat. Appl. Nos. 2001/0039430; 2002/0002360; 2003/0023259; 2003/0199809, and International patent application publication number WO 03/071926.The pre-amble of claim 1 is based an US 2004/0199121. The radially expandable access systems disclosed therein may include a pneumoperitoneum needle, an expandable sleeve component which is percutaneously introduced while positioned over the pneumoperitoneum needle, a cannula having a pneumostasis valve permanently affixed at its proximal end, and an obturator which is removably inserted into the cannula to form an expansion member for the sleeve. After the needle/sleeve assembly has been percutaneously introduced, and the peritoneal cavity insufflated in the case of laparoscopic procedures, the needle is removed from the sleeve, and the cannula/obturator assembly introduced through the sleeve. The sleeve, which initially has a diameter in the range of 2-3 mm, is thus expanded to a final diameter depending on the cannula size, which can be selected from 5 mm, 10 mm, or 12 mm. Use of the radially expandable access system has many advantages, including reduced trauma to the patient and the ability to replace a cannula with a larger diameter cannula through a previously introduced sleeve.

While the radially expandable access system represents a substantial advance over conventional trocars, the need and desire exists for improved radially expandable access systems, component kits for such systems, and methods for reconstructing and reusing such systems.

### SUMMARY

The present disclosure relates to access systems which may be percutaneously or otherwise introduced while in a narrow diameter configuration and, which after introduction, may be radially expanded to accommodate passage of larger diameter surgical instruments therethrough.

According to an aspect of the present disclosure, an access system is provided, which includes a radially expandable sleeve component, for use with the access system. The sleeve component includes a handle having a passage therethrough; and a sleeve body having a proximal end connected to the handle, a distal end, and an axial lumen aligned with the passage of the handle, the sleeve body having a length. The sleeve body is constructed from a radially expandable braid, wherein the braid is formed of a mesh of non-elastic filaments which axially shortens the length of the sleeve body as the sleeve body is radially expanded. The access system further includes a cannula tube having a proximal end, a distal end, and a lumen extending therethrough. The cannula tube is sized for reception in the passage of the handle of the radially expandable sleeve component. The cannula tube has a length which is shorter than the length of the sleeve body when the cannula tube is fully inserted into the sleeve body of the radially expandable sleeve component.
When the cannula tube is fully inserted into the sleeve body of the radially expandable sleeve component the distal end of the sleeve body extends beyond the distal end of the cannula tube. The distal end of the sleeve body may be flared radially outward.

The radially expandable sleeve may further include a sheath substantially encasing the sleeve body.

It is contemplated that the flared distal end of the sleeve body facilitates withdrawal of instruments from the radially expandable sleeve component.

The sheath may encase the sleeve body along at least a portion of the length thereof.

Desirably, the sheath maintains the flared distal end of sleeve body in a radially unexpanded condition. It is contemplated that the flared distal end of the sleeve body takes form upon removal of the sheath therefrom.

According to yet another aspect of the present disclosure, an access system is provided. The access system includes a radially expandable sleeve component including a handle having a passage therethrough; and a sleeve body having a proximal end connected to the handle, a distal end, and an axial lumen aligned with the passage of the handle, the sleeve body having a length. The distal end of the sleeve body tapers radially inward. The access system further includes a cannula tube having a proximal end, a distal end, and a lumen extending therethrough. The cannula tube is sized to be received in the passage of the handle of the radially expandable sleeve component. The cannula tube has a length which is shorter than the length of the sleeve body when the cannula tube is fully inserted into the sleeve body of the radially expandable sleeve component so that the tapered distal end of the sleeve body engages an instrument inserted into the radially expandable sleeve component.

The access systems of the present disclosure may further include an obturator removably receivable in the lumen of the cannula tube. The obturator has a tapered distal end which extends distally from the distal end of the cannula tube when the obturator is disposed in the lumen of the cannula tube. The access systems may further include a pneumoperitoneum needle including a tubular needle; and an internal stylet removably receivable within the tubular needle.

When the cannula tube is fully inserted into the sleeve body of the radially expandable sleeve component the flared distal end of the sleeve body extends beyond the distal end of the cannula tube.

The radially expandable sleeve further may include a sheath encasing the sleeve body along at least a portion of the length thereof. The sheath desirably maintains the radially inward tapered distal end of the sleeve body in the radially tapered condition. In use, the radially inward tapered distal end of the sleeve body radially expands upon removal of the sheath therefrom.

Other objects and features of the present disclosure will become apparent from consideration of the following description taken in conjunction with the accompanying drawings.

### BRIEF DESCRIPTION OF THE DRAWINGS

By way of example only, embodiments of the radially expandable access system of the present disclosure, will be described with reference to the accompanying drawings, in which:

FIG. 1 is a side view of a radially expandable sleeve component of the access system according to the present disclosure, including a removable sheath encasing a tubular braid portion thereof;

FIG. 1A is a longitudinal cross-sectional view of the radially expandable sleeve component of FIG. 1;

FIG. 2 is a side view of the radially expandable sleeve component of FIG. 1 with the sheath removed from the tubular braid portion thereof;

FIG. 3 is a side view of a prior art pneumoperitoneum needle component for use with the radially expandable sleeve component of FIGS. 1 and 2;

FIG. 4 is a side view of a prior art cannula assembly for use with the radially expandable sleeve component of FIGS. 1 and 2, shown with the cannula body, cannula hub, and valve cap removed or separated from each other, and further shown with the valve cap in partial section;

FIG. 5 is a side view of a prior art obturator component for use with the radially expandable sleeve component of FIGS. 1 and 2, and cannula assembly of FIG. 3;

FIG. 6 is a side view of the radially expandable sleeve component of FIGS. 1 and 2 having the cannula assembly of FIG. 3 operatively associated therewith and with the valve cap of the cannula assembly and the handle of the radially expandable sleeve component shown in partial section;

FIG. 7 is a side view of the radially expandable sleeve component of FIGS. 1 and 2 having the cannula assembly of FIG. 3 operatively associated therewith and a surgical instrument extending therethrough, with the valve cap of the cannula assembly and the handle of the radially expandable sleeve component being shown in partial section;

FIG. 7A is a cross-sectional side view of the radially expandable sleeve component of FIGS. 1 and 2 having the cannula assembly of FIG. 3 operatively associated therewith and a surgical instrument extending therethrough; and

FIGS. 8-13 illustrate use of the radially expandable sleeve component of FIGS. 1 and 2 in providing access to a patient's abdomen.

### DETAILED DESCRIPTION OF PREFERRED EMBODIMENTS

The access system of the present disclosure is useful for forming and enlarging percutaneous penetrations into a variety of target locations within a patient's body for a multiplicity of purposes. Such purposes include drainage, intra-organ drug administration, feeding, perfusion, aspiration, and the like, most usually being the introduction of viewing scopes and surgical instruments for use in minimally invasive surgical procedures, such as laparoscopic procedures, thoracoscopic procedures, arthroscopic procedures, endoscopic procedures, and the like. In addition to percutaneous procedures, the access system of the present disclosure will find use in hysteroscopic, colonoscopic, and other procedures where access is established through existing body orifices.

The access systems of the present disclosure are particularly valuable in percutaneous procedures since they will create a very small initial penetration, usually being below about 5 mm, more usually being below about 4 mm, frequently being below about 3.5 mm, and preferably being 3 mm or below. The penetration will be subsequently enlarged to a desired final size, usually having a final diameter in the range from about 5 mm to 15 mm, more usually being from about 5 mm to 12 mm, and typically being from about 5 mm to 10 mm. The enlarged penetration will define an access lumen from the outside of the patient's body to the desired internal location, and it is a particular advantage of the present disclosure that the diameter of the access lumen may be changed as will be described in more detail hereinafter. In non-percutaneous procedures, the access system is valuable since it is capable of passing through the existing body orifice in its narrow-diameter configuration and be subsequently expanded with minimum discomfort and trauma to the patient.

The access system of the present disclosure includes a number of individual components that may be assembled into different size configurations. The assembled components may also be disassembled after use, and the components selectively sterilized or replaced prior to reassembling the access system for fiuther use with a different patient. The different components and component assemblies and subassemblies will be described in greater detail below.

Sterilization of the components of the trocar system disclosed herein may be accomplished by any suitable conventional sterilization technique, including heat, e.g., steam and autoclaving; chemical treatment, e.g., ethylene oxide exposure; radiation, and the like. After use, reusable components will be washed to remove blood and other contaminating substances and then sterilized, preferably by exposure to steam. Disposable components will usually be radiation sterilized in their packages prior to distribution. Thus, disposable components will usually be ready to use out of the package.

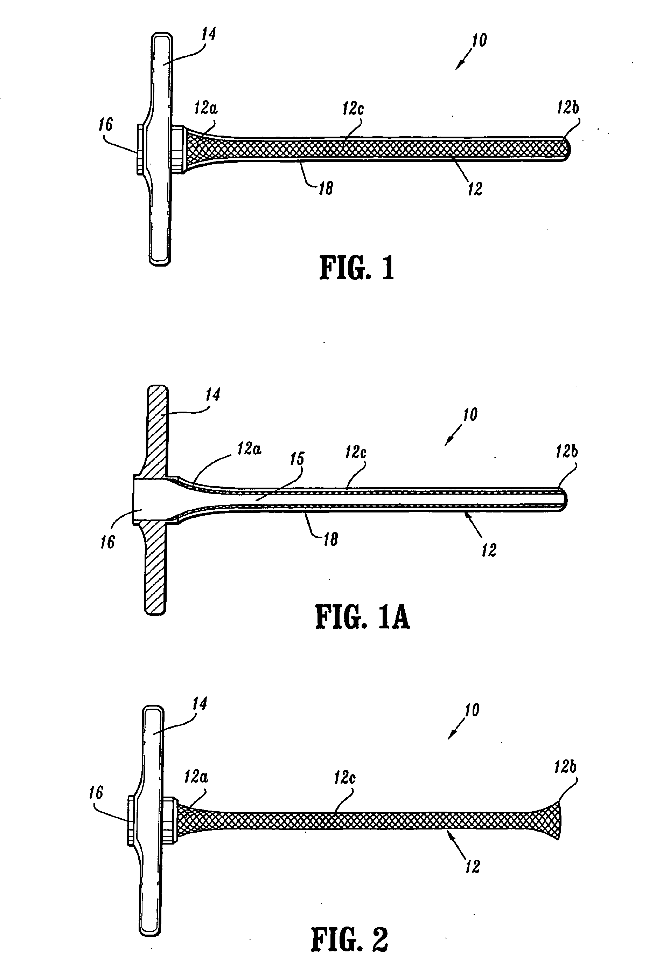

Referring initially to FIGS. 1, 1A and 2, wherein like reference numerals identify similar or identical structural elements, a radially expandable sleeve component or trocar seal, according to an embodiment of the present disclosure, for use as part of an access system, is generally designated as 10. As used herein, the term "distal" refers to that portion of the tool, or component thereof which is further from the user while the term "proximal" refers to that portion of the tool or component thereof which is closer to the user.

As seen in FIGS. 1,1A and 2, sleeve component 10 includes a sleeve body 12 defining a lumen 15 (see FIG. 1A) from a proximal end 12a to a distal end 12b thereof, and a handle 14 operatively connected to proximal end 12a of sleeve body 12. Preferably, sleeve body 12 is constructed from a radially expandable braid, desirably inelastic, having an inner diameter of about 2 mm and an outer diameter of about 3.5 mm. Handle 14 includes a passage 16 (see FIG. 1A) formed therethrough, which passage 16 is substantially aligned with the lumen of sleeve body 12. Desirably and typically a connector (not shown) is provided about passage 16 for selectively engaging a complementary connector provided on a cannula assembly 40. For example, the complementary connectors may take the form of threads, bayonet fittings, and the like. As will be described in greater detail below, passage of an expansion assembly therethrough causes radial expansion of sleeve body 12, typically to a final diameter of 5 mm, 10 mm, or 12 mm. Radially expandable sleeve 10 may be constructed in accordance with the details set forth in U.S. Pat. No. 5,431,676.

As seen in FIG. 2, distal end 12b of sleeve body 12 is flared radially outward. In particular, sleeve body 12 includes an intermediate portion 12c having a uniform diameter along substantially the entire length thereof, and a distal end 12b having a diameter which is larger than the diameter of intermediate portion 12c.

As seen in FIG. 1, a sheath 18 encases and/or otherwise covers sleeve body 12. Sheath 18 extends the entire length of sleeve body 12. Desirably, sheath 18 is fabricated from a plastic or elastomeric material, e.g., polyurethane, tetrafluorethylene, fluorinated ethylene-propylene, or the like. Desirably, sheath 18 will be weakened along an axial line (as by a pair of thin or weakened axial grooves or lines (not shown)) to facilitate splitting of sheath 18 at some point during the procedure. As described in more detail hereinafter, the axial grooves enable sheath 18 to be divided or split along the length thereof, as cannula assembly 40 is received in the lumen of sleeve body 12, and thus allow sleeve body 12 to radially expand.

Additionally, as seen in FIG. 1, sheath 18 helps to maintain flared distal end 12b closed (i.e., in a radially unexpanded condition) prior to introduction of the first surgical instrument. In other words, sheath 18 constricts distal end 12b such that distal end 12b has a diameter which is substantially equal to the diameter of intermediate portion 12c of sleeve body 12.

The braid of sleeve body 12 is formed as a mesh of individual non-elastic filaments (e.g., composed of polyamide fiber, stainless steel, or the like) so that radial expansion causes axial shortening of the braid. Additionally, the braid of sleeve body 12 may be constructed from round filaments, flat or ribbon filaments, square filaments, or the like. Non-round filaments may advantageously reduce the axial force required to provide radial expansion. The filament width or diameter will typically be from about 0.002 inches (0.05 mm) to about 0.25 inches (6.4 mm), usually being from about 0.005 inches (0.1 mm) to about 0.010 inches (0.3 mm).

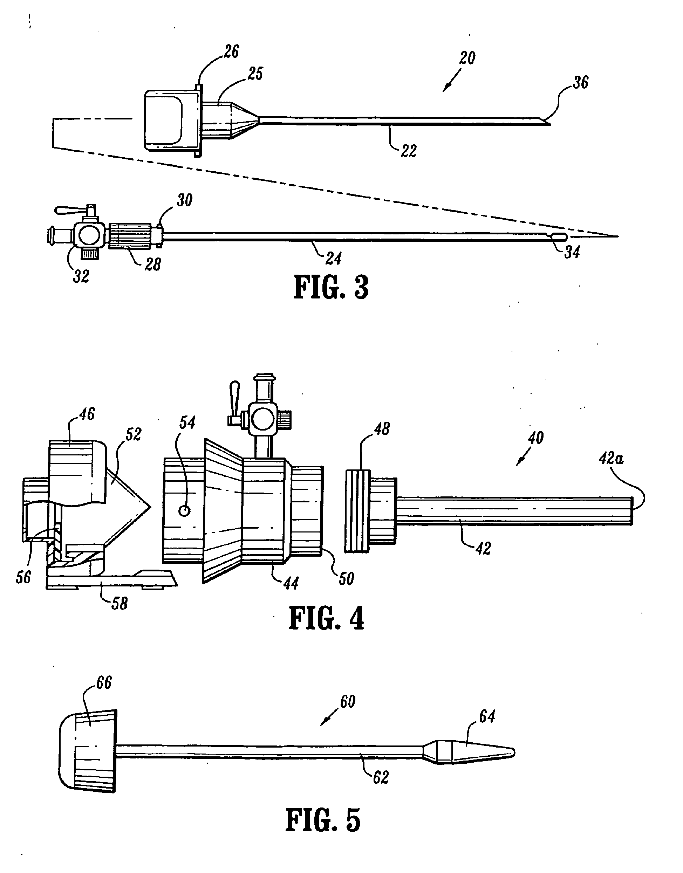

Turning now to FIG. 3, a pneumoperitoneum needle assembly, for use as part of an access system, is generally designated as 20. Pneumoperitoneum needle assembly 20 includes a tubular needle body 22, and a stylet 24 for operative engagement with tubular needle body 22. Tubular needle body 22 includes a hub 25, having a male bayonet connector 26 extending therefrom, provided at a proximal end thereof. Stylet 24 is spring-loaded in a connector 28 which is provided at a proximal end thereof. Connector 28 includes a male bayonet fitting 30 which is receivably mounted in a female bayonet fitting (not illustrated) provided in hub 25 of needle body 22. Stylet 24 further includes an insufflation valve 32 provided at a proximal end thereof, and a port 34 formed in a distal end thereof Accordingly, insufflation gas, introduced through valve 32, is permitted to be released through port 34. In use, stylet 24 is to be mounted within tubular needle body 22 by way of bayonet fittings 30 of connector 28. The distal end of stylet 24 will extend from distal end 36 of needle body 22, and stylet 24 will retract into needle body 22 when needle body 22 is engaged against tissue, as described in more detail below.

Turning now to FIG. 4, a cannula assembly, for use as part of an access system, is generally designated as 40. Cannula assembly 40 includes a cannula tube 42, a cannula hub 44 connectable to cannula tube 42, and a valve cap 46 removably connectable to cannula hub 44. Cannula tube 42 includes a threaded connector 48 at a proximal end thereof which may be removably secured or connected to a fitting 50 provided at a distal end of cannula hub 44. Valve cap 46 desirably includes a pneumostasis valve element 52 and is configured to mate with a male bayonet fitting 54 provided at a proximal end of cannula hub 44. A second disk valve element 56 may be mounted in tandem with the pneumostasis valve element 52 to engage against an outer surface of a surgical instrument (not shown) when the surgical instrument is introduced through cannula assembly 40. Valve element 56 is generally sized for a relatively large instrument, e.g., an instrument having a diameter of about 12 mm. A reducing element 58 may be provided for reducing the size of the port of valve element 56 to accommodate relatively smaller instruments, e.g., instruments having a diameter of about 10 mm.

Turning now to FIG. 5, an obturator, for use as part of an access system, is generally designated as 60. Obturator 60 generally includes a shaft 62, a tapered distal end 64, and a handle 66. Obturator 60 is intended to be placed within a central lumen of cannula assembly 40 in order to form an expansion assembly for use as described below.

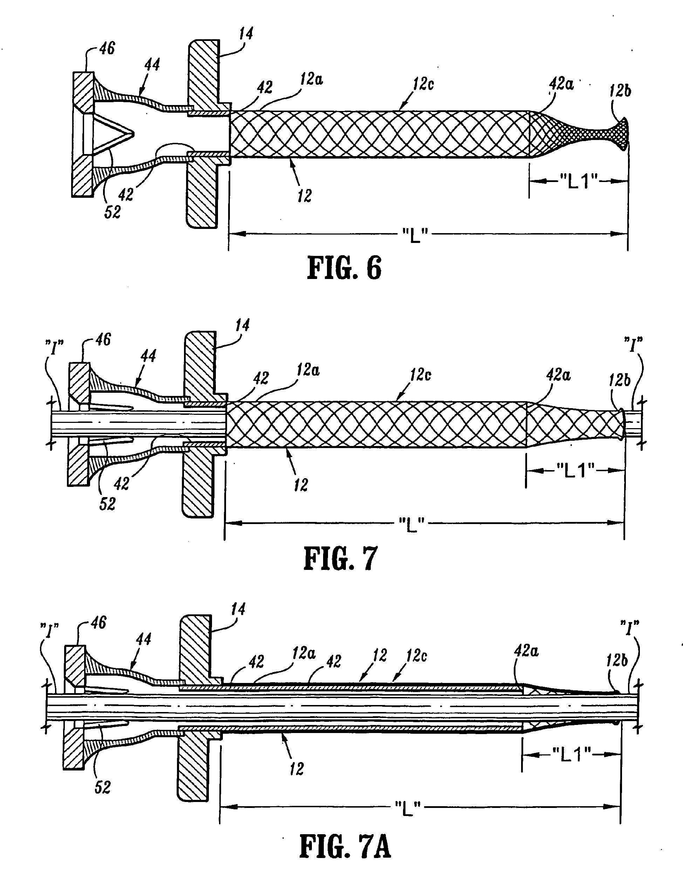

With reference now to FIG. 6, radially expandable sleeve component 10 is shown in operative association with cannula assembly 40. In particular, cannula tube 42 of cannula assembly 40 has been fully inserted into the lumen of sleeve body 12 of expandable sleeve component 10. Desirably, sleeve body 12 has a length "L" which is greater then the length of cannula tube 42 when cannula tube 42 has been fully inserted into expandable sleeve component 10. In this manner, distal end 12b of sleeve body 12 extends distally beyond a distal edge 42a of cannula tube 42. Desirably, length "L" of sleeve body 12 is such that flared distal end 12b thereof is spaced an axial distance "L1" from distal edge 42a of cannula tube 42 when cannula tube 42 is fully inserted into expandable sleeve component 10.

With reference to FIG. 7, flared distal end 12b of sleeve body 12 effectively forms and/or acts as an instrument seal against the surface of an instrument "I" introduced into and extending through cannula tube 42 of cannula assembly 40 and sleeve body 12 of expandable sleeve component 10. Flared distal end 12b of sleeve body 12 is provided in order to facilitate removal of instrument "I" from cannula assembly 40 and, in particular, from sleeve body 12 of expandable sleeve component 10.

While distal end 12b of sleeve body 12 is preferably provided with a flare, it is within the scope of the present disclosure, that distal end 12b of sleeve body 12 does not have to include a flare or the like in order to create and/or act as a instrument seal.

Desirably, as seen in FIGS. 4, 6 and 7, pneumostasis valve element 52 of cannula hub 44 may take the form of a duck bill or "zero" valve. Valve element 52 may include two planar tapering portions which intersect at their distal ends to define an abutment face. The planar tapering portions may each include one or more inwardly directed, longitudinally oriented ribs to facilitate passage of instrument "I". The abutment face permits passage of instrument "I" through valve element 52, but in the absence of instrument "I", and particularly when cannula assembly 40 is inserted into an insufflated body cavity, the abutment face forms a gas-tight seal that isolates the insufflation cavity from the ambient surroundings. Valve element 52 also includes at least one, preferably two, reinforcing ribs (not shown) to stabilize valve element 52. The ribs are positioned to engage instrument "I" to guide instrument "I" through the slit of valve element 52 and prevent piercing of valve element 52 by the tip of instrument "I". Reference may be made to U.S Patent 5,603,702 for a more detailed discussion of a valve element.

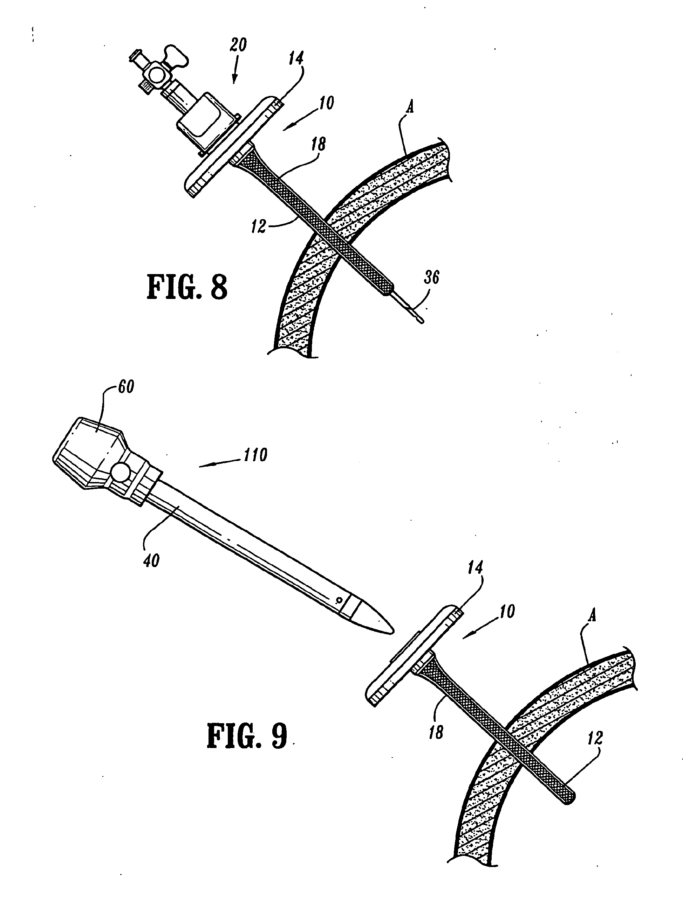

Referring now to FIGS. 8-13, use of radially expandable sleeve component 10, in an access system, will be described in detail. Initially, as seen in FIG. 8, a radially expandable sleeve component 10, having a pneumoperitoneum needle 20 inserted therein, is introduced through a patient's abdomen "A" (or other body location) by engaging sharpened distal end 36 of needle 20 against the tissue and advancing the assembly (e.g., expandable sleeve component 10 operatively coupled with needle 20) until sleeve body 12 of radially expandable sleeve component 10 extends across the tissue.

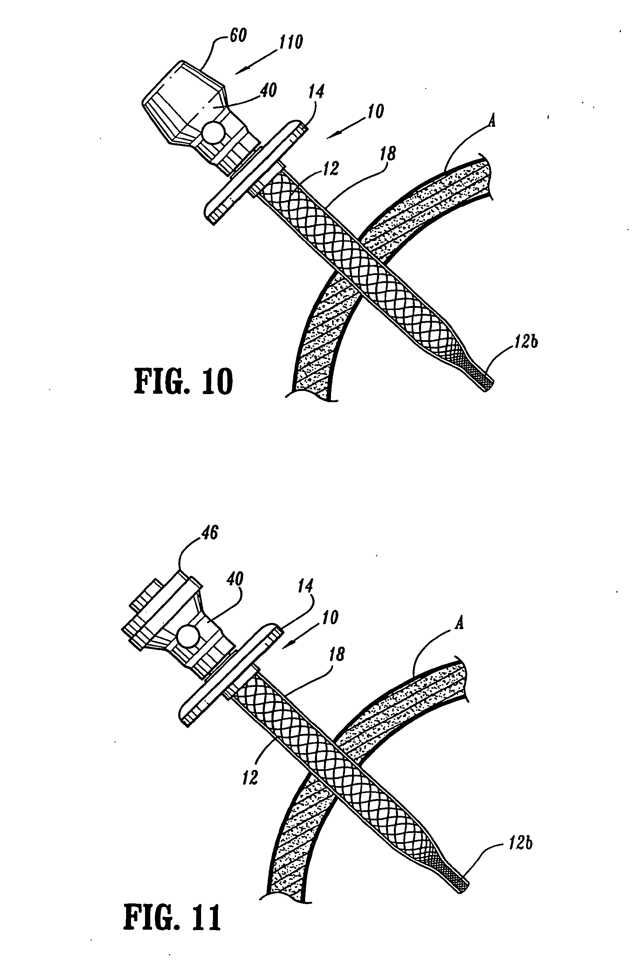

As seen in FIG. 9, needle 20 is removed from expandable sleeve component 10 and an expansion assembly 110, including cannula assembly 40 having obturator 60 operatively associated therewith, is introduced through radially expandable sleeve component 10. Introduction of expansion assembly 110 into radially expandable sleeve component 10 results in radial expansion of sleeve body 12 (see FIG. 10). In so doing, sheath 18 is divided or split along the length of the axial grooves (not shown). Additionally, insertion of expansion sleeve 110 into expandable sleeve component 10 to radially expand sleeve body 12 results in axial shortening of sleeve body 12 to thereby help anchor expansion assembly 110 in place and to help seal the exterior of expansion assembly 110 against the tissue.

As described above, when expansion assembly 110 is fully inserted into radially expandable sleeve component 10, distal edge 42a of cannula tube 42 does not extend beyond distal end 12b of sleeve body 12. Desirably, sleeve body 12 has a length "L" sufficient that when expansion assembly 110 is fully inserted into sleeve body 12 of expandable sleeve component 10, obturator 60 and cannula tube 40 do not radially expand distal end 12b of sleeve body 12 and, thus, do not split open a distal end of sheath 18.

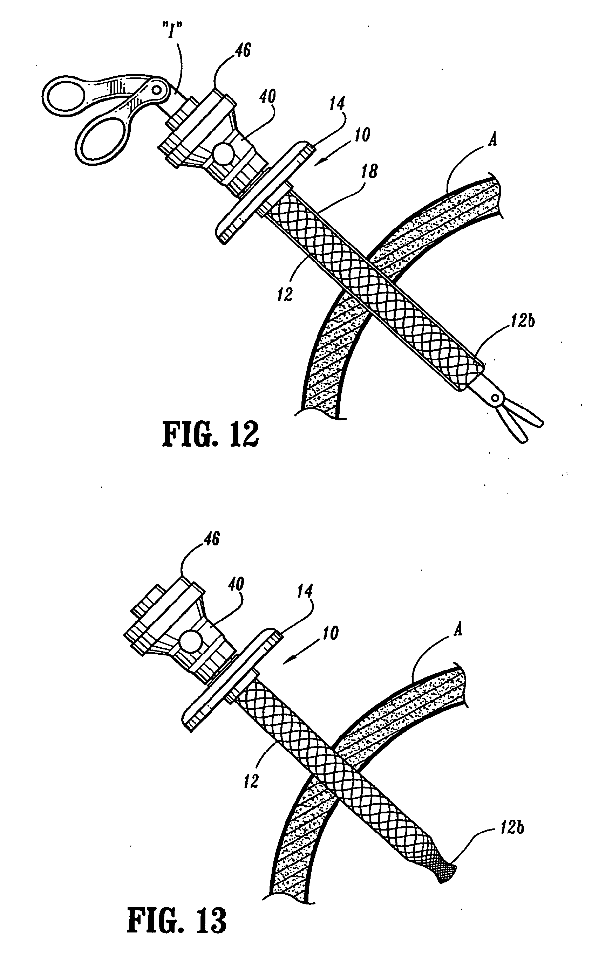

As seen in FIG. 11, obturator 60 may then be removed from cannula assembly 40 and radially expandable sleeve 40, leaving an access channel through abdominal wall "A". With obturator 60 removed, as seen in FIG. 12, a surgical instrument "I" (e.g., surgical graspers, staplers, tackers, fastener appliers, etc.) may be introduced, through cannula assembly 40 and radially expandable sleeve component 10, into the abdominal cavity. Desirably, instrument "I" has a length such that an end effector of instrument "I" is extendable beyond distal edge 42a of cannula tube 42 and beyond distal end 12b of sleeve body 12 of expandable sleeve component 10. Introduction of instrument "I" through distal end 12b of sleeve body 12 results in radial expansion of the same and, thus, in the dividing and/or splitting of the distal end of sheath 18. With sheath 18 divided along its entire length, it is now possible, if desired, to withdraw and remove sheath 18 from between the surface of the incision and sleeve body 12 of expandable sleeve component 10, as seen in FIG. 13.

With reference to FIG. 12, distal end 12b of sleeve body 12 acts as an instrument seal against the outer surface of instrument "I", thereby reducing the escape or passage of insufflation fluid through cannula tube 42. Such a fluid-tight seal is a particular advantage in laparoscopic procedures.

With reference to FIG. 13, following use of surgical instrument "I" in performing the surgical procedure, surgical instrument "I" may be removed and/or withdrawn from expansion assembly 110 and radially expandable sleeve component 10. Flared distal end 12b of sleeve body 12 facilitates the removal and/or withdrawal of surgical instrument "I" from expansion assembly 110 and radially expandable sleeve component 10. Additionally, flared distal end 12b of sleeve body 12 may act like a funnel to facilitate removal and/or retraction of a tissue or organ specimen from the abdominal cavity.

The above is a complete description of preferred embodiments of the disclosure The above description should not be taken as limiting the scope of the invention which is defined by the appended claims.

## Claims

1. An access system, comprising:
a radially expandable sleeve component (10), for use with the access system, the sleeve component comprising:
a handle (14) having a passage (16) therethrough; and
a sleeve body (12) having a proximal end (12a) connected to the handle, a distal end (12b), and an axial lumen (15) aligned with the passage of the handle, the sleeve body having a length (L), the sleeve body being constructed from a radially expandable braid, wherein the braid is formed of a mesh of non-elastic filaments which axially shortens the length of the sleeve body as the sleeve body is radially expanded; the access system further comprising :
a cannula tube (42) having a proximal end, a distal end, and a lumen extending therethrough, the cannula tube being sized to be received in the passage of the handle of the radially expandable sleeve component;
the system **characterised by**
the cannula tube having a length which is shorter than the length of the sleeve body when the cannula tube is fully inserted into the sleeve body of the radially expandable sleeve component so that when the cannula tube is fully inserted into the sleeve body of the radially expandable sleeve component the distal end of the sleeve body extends beyond the distal end of the cannula tube.

2. The access system of claim 1, wherein the distal end of the sleeve body is flared radially outward.

3. The access system of claim 2, wherein the flared distal end of the sleeve body facilitates withdrawal of surgical instruments (I) from the radially expandable sleeve component.

4. The access system of claim 2 or 3,
wherein the length of the sleeve body is such that the flared distal end thereof is spaced an axial distance (L1) from a distal edge (42a) of the cannula tube.

5. The access system according to any one of the preceding claims, wherein the radially expandable sleeve further includes a sheath (18) encasing the sleeve body along at least a portion of the length thereof.

6. The access system according to claim 5 when dependent on claim 2, 3 or 4, wherein the sheath maintains the flared distal end of sleeve body in a radially unexpanded condition.

7. The access system according to claim 6, wherein the flared distal end of the sleeve body takes form upon removal of the sheath therefrom.

8. The access system of any one of the preceding claims,
wherein the sleeve body radially expands upon insertion of the cannula tube.

9. The access system of any one of the preceding claims, further comprising an obturator (60) removably receivable in the lumen of the cannula tube, the obturator having a tapered distal end (64) which extends distally from the distal end of the cannula tube when the obturator is disposed in the lumen of the cannula tube.

10. The access system of any one of the preceding claims, further comprising a pneumoperitoneum needle assembly (20) including:
a needle having a tubular body (22); and
an internal stylet (24) removably receivable within the tubular body.

## Patentansprüche

1. Zugangssystem, umfassend:
ein radial erweiterbares Hülsenteil (10) zur Verwendung mit dem Zugangssystem, wobei das Hülsenteil umfasst:
einen Griff (14) mit einem Durchgang (16) **dadurch**, und
einen Hülsenkörper (12) mit einem proximalen Ende (12a), das an dem Griff befestigt ist, einem distalen Ende (12b) und einem axialen Lumen (15), das mit dem Durchgang des Griffs fluchtet, wobei der Hülsenkörper eine Länge (L) hat, wobei der Hülsenkörper aus einem radial erweiterbaren Geflecht aufgebaut ist, wobei das Geflecht aus einem Netz aus nichtelastischen Filamenten gebildet ist, das die Länge des Hülsenkörpers verkürzt, wenn der Hülsenkörper radial erweitert wird, wobei das Zugangssystem ferner umfasst:
eine Kanülenröhre (42) mit einem proximalen Ende, einem distalen Ende und einem Lumen, das sich **dadurch** erstreckt, wobei die Kanülenröhre bemessen ist, um in dem Durchgang des Griffs des radial erweiterbaren Hülsenteils aufgenommen zu werden,
wobei das System **dadurch gekennzeichnet ist, dass**
die Kanülenröhre eine Länge aufweist, die kürzer als die Länge des Hülsenkörpers ist, wenn die Kanülenröhre vollständig in den Hülsenkörper des radial erweiterbaren Hülsenteils eingesetzt ist, so dass, wenn die Kanülenröhre vollständig in den Hülsenkörper des radial erweiterbaren Hülsenteils eingesetzt ist, sich das distale Ende des Hülsenkörpers über das distale Ende der Kanülenröhre hinaus erstreckt.

2. Zugangssystem nach Anspruch 1, wobei das distale Ende des Hülsenkörpers radial nach außen aufgeweitet ist.

3. Zugangssystem nach Anspruch 2, wobei das aufgeweitete distale Ende des Hülsenkörpers ein Zurückziehen chirurgischer Instrumente (I) aus dem radial erweiterbaren Hülsenteil erleichtert.

4. Zugangssystem nach Anspruch 2 oder 3,
wobei die Länge des Hülsenkörpers derart ist, dass dessen aufgeweitetes distales Ende um einen axialen Abstand (L1) von einer distalen Kante (42a) der Kanülenröhre beabstandet ist.

5. Zugangssystem nach einem der vorstehenden Ansprüche,
wobei die radial erweiterbare Hülse ferner eine Schutzhülle (18) enthält, die den Hülsenkörper entlang zumindest einem Abschnitt seiner Länge umgibt.

6. Zugangssystem nach Anspruch 5, abhängig von Anspruch 2, 3 oder 4, wobei die Schutzhülle das aufgeweitete distale Ende des Hülsenkörpers in einem radial unerweiterten Zustand hält.

7. Zugangssystem nach Anspruch 6, wobei das aufgeweitete distale Ende des Hülsenkörpers nach einem Entfernen der Schutzhülle davon Form annimmt.

8. Zugangssystem nach einem der vorstehenden Ansprüche,
wobei sich der Hülsenkörper nach einem Einsetzen der Kanülenröhre radial erweitert.

9. Zugangssystem nach einem der vorstehenden Ansprüche, ferner umfassend einen Obturator (60), der herausnehmbar in dem Lumen der Kanülenröhre aufnehmbar ist, wobei der Obturator ein sich verjüngendes distales Ende (64) aufweist, das sich distal von dem distalen Ende der Kanülenröhre erstreckt, wenn der Obturator in dem Lumen der Kanülenröhre angeordnet ist.

10. Zugangssystem nach einem der vorstehenden Ansprüche, ferner umfassend eine Pneumoperitoneum-Nadelanordnung (20) umfasst, die
eine Nadel mit einem röhrenförmigen Körper (22) und
ein inneres Stilett (24) enthält, das herausnehmbar innerhalb des röhrenförmigen Körpers aufnehmbar ist.

## Revendications

1. Système d'accès, comprenant:
un composant de manchon radialement expansible (10), pour utilisation avec le système d'accès, le composant de manchon comprenant:
une poignée (14) ayant un passage (16) à travers celle-ci; et
un corps de manchon (12) ayant une extrémité proximale (12a) reliée à la poignée, une extrémité distale (12b), et une lumière axiale (15) alignée avec le passage de la poignée, le corps de manchon ayant une longueur (L), le corps de manchon étant réalisé à partir d'une tresse radialement expansible, où la tresse est réalisée en une maille de filaments non-élastiques qui raccourcit axialement la longueur du corps de manchon lorsque le corps de manchon est expansé radialement; le système d'accès comprenant en outre:
un tube de canule (42) ayant une extrémité proximale, une extrémité distale et une lumière s'étendant à travers celui-ci, le tube de canule étant dimensionné pour être reçu dans le passage de la poignée du composant de manchon radialement expansible;
le système étant **caractérisé par**
le tube de canule ayant une longueur qui est plus courte que la longueur du corps de manchon lorsque le tube de canule est entièrement inséré dans le corps de manchon du composant de manchon radialement expansible de sorte que lorsque le tube de canule est entièrement inséré dans le corps de manchon du composant de manchon radialement expansible, l'extrémité distale du corps de manchon s'étend au-delà de l'extrémité distale du tube de canule.

2. Système d'accès selon la revendication 1, dans lequel l'extrémité distale du corps de manchon est évasée radialement vers l'extérieur.

3. Système d'accès selon la revendication 2, dans lequel l'extrémité distale évasée du corps de manchon facilite le retrait des instruments chirurgicaux (I) du composant de manchon radialement expansible.

4. Système d'accès selon la revendication 2 ou 3, dans lequel la longueur du corps de manchon est telle que son extrémité distale évasée est espacée d'une distance axiale (L1) d'un bord distal (42a) du tube de canule.

5. Système d'accès selon l'une quelconque des revendications précédentes, dans lequel le manchon radialement expansible comprend en outre une gaine (18) renfermant le corps de manchon le long d'au moins une portion de sa longueur.

6. Système d'accès selon la revendication 5, lorsqu'elle dépend de la revendication 2, 3 ou 4, dans lequel la gaine maintient l'extrémité distale évasée du corps de manchon dans un état radialement non expansé.

7. Système d'accès selon la revendication 6, dans lequel l'extrémité distale évasée du corps de manchon prend forme lors du retrait de la gaine de celle-ci.

8. Système d'accès selon l'une quelconque des revendications précédentes, dans lequel le corps de manchon s'expanse radialement lors de l'insertion du tube de canule.

9. Système d'accès selon l'une quelconque des revendications précédentes, comprenant en outre un obturateur (60) pouvant être reçu amoviblement dans la lumière du tube de canule, l'obturateur ayant une extrémité distale retrécie (64) qui s'étend distalement depuis l'extrémité distale du tube de canule lorsque l'obturateur est disposé dans la lumière du tube de canule.

10. Système d'accès selon l'une quelconque des revendications précédentes, comprenant en outre un ensemble d'aiguille de pneumopéritoine (20) incluant:
une aiguille ayant un corps tubulaire (22); et
un stylet interne (24) pouvant être reçu amoviblement dans le corps tubulaire.
